(19) Europäisches Patentamt
European Patent Office
Office européen des brevets

(11) **EP 2 425 835 A1**

(12) **EUROPEAN PATENT APPLICATION**

(43) Date of publication:
**07.03.2012 Bulletin 2012/10**

(51) Int Cl.:
*A61K 31/205* (2006.01)   *A61K 31/216* (2006.01)
*A61K 31/353* (2006.01)   *A61P 35/00* (2006.01)

(21) Application number: **10173254.3**

(22) Date of filing: **18.08.2010**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**
Designated Extension States:
**BA ME RS**

(71) Applicant: **Grindeks, a joint stock company
1057 Riga (LV)**

(72) Inventors:
• **Kalvins, Ivars
Riga 1006 (LV)**

• **Stonans, Ilmars
Riga 1057 (LV)**
• **Sestakova, Irina
Riga 1006 (LV)**
• **Domracova, Ilona
Riga 1006 (LV)**
• **Jascenko, Elina
Riga 1006 (LV)**
• **Bridane, Veronika
Riga 1006 (LV)**
• **Kanepe, Iveta
Riga 1006 (LV)**

(54) **A new medical use of 3-(2,2,2-trimethylhydrazinium) propionate dihydrate and natural flavonoid derivatives**

(57) The present invention relates to highly effective treatment of cancer with 3-(2,2,2-trimethylhydrazinium) propionate dihydrate or its pharmaceutically acceptable salts and naturally occuring flavonoids, preferably Caffeic acid phenethyl ester and (+)-Catechin.

EP 2 425 835 A1

**Description**

Technical Field

The present invention relates to a novel composition of Meldonium dihydrate or its pharmaceutically acceptable salts and natural flavonoid derivatives for potentiating antitumour effect and for treating tumours; in particular it relates to an unexpected synergism of a combination of a natural flavonoid derivatives and an antiischemic agent Meldonium dihydrate in the treatment of cancer; and to processes for preparing the composition and its use for chemotherapy of tumours, especially malignant tumours.

Background Art

More than 22 million people are diagnosed with cancer every year. It is estimated that there will be 16 million new cases every year by 2020. Cancer causes 7 million deaths every year - or 12.5% of deaths worldwide.

Therefore, a great number of investigations, developments and research works have been carried out to find out anticancer agents, which can be used in chemotherapy of malignant tumours. There are known various anticancer agents and results of cancer treatment improve with every year, nevertheless the amount of effective dose of anticancer agent for treating is still high.

In nowadays a big role in anticancer experiments and in treating cancer take natural compounds. Flavonoids are one of them, which are naturally occurring anticancer agents.

Flavonoids are most commonly known for their antioxidant activity. However, it is now known that the health benefits they provide against cancer and heart disease are the result of other mechanisms.

There are many naturally founding flavonoids. Some of such are phenolic compounds - Caffeic Acid Phenethyl Ester (CAPE) - phenolic compound contained in propolis and a phenolic antioxidant abundant in certain fruits - (+)-Catechin.

The biological activities of propolis and CAPE have been studied, and it has been shown that CAPE has an antitumoral activity. Patent US 5008441 (UNIV COLUMBIA, published 16.04.1991) discloses a method for producing caffeic acid phenethyl ester (CAPE) and a method which substantially inhibits the growth of transformed cells without substantially inhibiting the growth of normal cells. This process comprises treating a population of cells with an effective inhibiting amount of caffeic acid phenethyl ester so as to substantially inhibit the growth of the transformed cells. Transformed cells are human breast carcinoma cells, human melanoma cells or colon or renal carcinoma cells.

(+)-Catechin (Ca) produces antimutagenic effects and the data presented in this study show that intestinal tumors in Min/+ mice are prevented by dietary administration of the flavonoid, (+)-catechin. MICHAEL J. (+)-Catechin inhibits intestinal tumor formation and suppresses focal adhesion kinase activation in the Min/+ mouse. Cancer Research. 2001, Jan, vol.61, no.1, p.118-125.

From prior art it has been known that there is a co-administration of CAPE and (+)-catechin in prevention of cancer.

Cell migration is essential for invasive and metastatic phenotypes of cancer cells. Potential chemopreventive agents of cancer-sulindac sulphide, caffeic acid phenethyl ester (CAPE), curucumin, and (+)-catechin - have been reported to interfere with several types of intracellular signals. Our observations raise the possibility that these agents, except for (+)-catechin, can be applied not only as chemopreventive agents but also as anti-metastatic therapy. SHIGEOKA, YASUSHI, IGISHI. Sulindac sulfide and caffeic acid phenethyl ester suppress the motility of lung adenocarcinoma cells promoted by transforming growth factor-beta through Akt inhibition. CANCER RESEARCH NAD CLINICAL ONCOLOGY. 2004 March, vol.130, no.3, p.146-152.

3- (2,2,2-Trimethylhydrazinium) propionate dihydrate is known as compound with cardioprotective properties (this substance being known under its International Nonproprietary Name of Meldonium). 3- (2,2,2-Trimethylhydrazinium) propionate is disclosed in US 4481218 (INST ORGANICHESKOGO SINTEZA) 06.11.1984

Meldonium dihydrate doesn't possess anticancer activity; there are no data, that it could have it.

Disclosure of Invention

Technical problem

In malignant tumour treatment different kind of pharmaceutical compositions are used, including compositions of different natural compounds, but just only partial remission of malignant tumour was obtained.

Technical solution

The present invention is directed to a compound for treating a tumour, especially malignant tumour in an individual who need such treatment, comprising the administration to said individual of a pharmaceutically effective dose of CAPE and (+)-Catechin and Meldonium dihydrate.

While attempting to develop a pharmaceutical composition for malignant tumour treatment having as essentially lower toxicity, we unexpectedly found that doses adequate to the therapeutic ones used in the clinical use of pharmaceutical compositions containing CAPE, (+)-Catechin and Meldonium dihydrate in a pharmaceutical composition leads to advantageous effects.

The use of a composition of Meldonium, CAPE and (+)-Catechin, increases lifespan.

Therefore, the synergistic effect on CAPE, (+)-Catechin and Meldonium dihydrate is unexpected and surprising. Mode(s) for Carrying Out Invention

Experimental example

Experiments in vivo were carried out on mice sarcoma S-180 ascites form.

The anti-tumour activity of the pharmaceutical composition of CAPE, (+)-Catechin and Meldonium dihydrate was determined by introducing them into ICR mice of initial weight 20-23 g during a fortnight. Throughout the experiment, the mice were kept under standard laboratory conditions at a temperature of $22\pm2$ °C, relative humidity $55 \pm15\%$, ventilation - 15-20 changes of air amount/hour and a 12 hours light/darkness cycle, and were fed with standard laboratory animal food.

Mice sarcoma S-180 transplanted intraperitoneally to ICR mice in amount $5 \times 10^6$ cell/mouse.

Then, 24 hours later, orally administration of compounds was started once a day at the 1st, 3rd, 5th, 7th, 9th, 11th and 13th days.

Flavonoids - Caffeic acid phenethyl ester was administered in a dose 27 mg/kg and (+)-Catechin was administered in a dose 300 mg/kg and they were added to a food. Meldonium dihydrate was administered into a drinking water and was given day and night in a dose 200 mg/kg; animals drank water for 13 days.

We determined Lifespan, which in the control's group is $24 \pm 2$ days. If the effect of compound is $\geq 25\%$, than it can be taken as active.

The percentage of increase lifespan (ILS%) was calculated according the formula:

$$ILS\% = \frac{T - C}{C} \times 100 \quad ,$$

wherein T represents the mean survival time of treated animals; C represents the mean survival time of the control group.

Composition of Caffeic acid phenethyl ester and (+)-Catechin and Meldonium dihydrate.

**DAY 13**

Table 1

Lifespan of the pharmaceutical composition on a day 13th

To our surprise, using a pharmaceutical composition of (CAPE 27 mg/kg + Ca 300 mg/kg) + Meldonium dihydrate (200 mg/kg) it has very good results in Increase Lifespan (%) that is 54 %. *p <0.05 relative to CAPE+Ca (27 mg/kg + 300 mg/kg group), #p<0.05 relative to Meldonium dihydrate 200 mg/kg.

Formulation Example 1: Granule

Table 2

| | |
|---|---|
| Meldonium | 200 mg |
| CAPE | 300 mg |
| Catechin | 27 mg |
| Lactose | 440 mg |
| Corn starch | 513 mg |
| Hydroxypropylmethylcellulose | 20 mg |
| Total | 1500 mg |

Using the conventional procedure, the granules were prepared according to the above formula

Formulation Example 2: Tablet

Table 3

| | |
|---|---|
| Meldonium | 20 mg |
| CAPE | 30 mg |
| Catechin | 2.7 mg |
| Lactose | 120 mg |
| Crustalline cellulose | 35 mg |
| Magnesium stearate | 12 mg |
| Talc | 15.3 mg |
| Methylcellulose | 15 mg |

(continued)

| Total | 250 mg |
|---|---|

Using the convential procedure, the tablets each weighing 250 mg were prepared according to the above formula.

Formulation Example 3: Suppository

Table 4

| Meldonium | 200 mg |
|---|---|
| CAPE | 300 mg |
| Catechin | 27 mg |
| Witepsol W-35 | 973 mg |
| Total | 1500 mg |

Using the convential procedure, the suppositories each weighing 1500 mg was prepared according to the above formula.

Formulation Example 4: Parenteral solution

Table 5

| Meldonium | 200 mg |
|---|---|
| CAPE | 300 mg |
| Catechin | 27 mg |
| Sodium carbonate | 500 mg |
| Sodium hydroxide | 80 mg |
| Distilled water | (suitable amount) |
| Total | 10 ml (per ampule) |

Using the convential procedure, the parenteral solution was prepared according to the above formula.

Industrial Application

According to the invention, the pharmaceutical composition for treating malignant tumour which contains Meldonium dihydrate, Caffeic acid phenethyl ester and one of natural flavonoid derivative, preferably (+)-Catechin, in clinically efficacious amount might be manufactured in a standard pharmaceutical plant.

**Claims**

1.    A pharmaceutical composition comprising Meldonium (3-(2,2,2-trimethylhydrazinium)propionate or a pharmaceutically acceptable salt thereof and one derivative or two derivatives of natural flavonoids, selected from the group of caffeic acid phenethyl ester (CAPE) and (+)-catechin.

2.    A pharmaceutical composition according to claim 1, wherein the preferable ratio of the active ingredients Meldonium, caffeic acid phenethyl ester (CAPE) and catechin is 100:100:1 1 or 1:1:100.

3.    A pharmaceutical composition according to claim 1, wherein the preferable ratio of the active ingredients Meldonium, caffeic acid phenethyl ester (CAPE) and catechin is 20:20:1 or 1:1:20.

4.    A pharmaceutical composition according to claim 1, wherein the preferable ratio of the active ingredients Meldonium, caffeic acid phenethyl ester (CAPE) and catechin is 7:11:1 or 1:11:7.

5.    Use of pharmaceutical composition of any preceding claim as a medicament.

**6.** A pharmaceutical composition according to claims 1-4 for use in the treatment of a tumour.

**7.** Use of a pharmaceutical composition according to claims 1-4 for the manufacture of a medicament for administration simultaneously, sequentially or separately for treatment and/or prevention of a tumour.

Europäisches
Patentamt

European
Patent Office

Office européen
des brevets

# EUROPEAN SEARCH REPORT

Application Number

EP 10 17 3254

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2009/071585 A1 (GRINDEKS A JOINT STOCK COMPANY [LV]; CHEMCO VENTURES CYPRUS LTD [CY];) 11 June 2009 (2009-06-11) | 1-5 | INV.<br>A61K31/205<br>A61K31/216 |
| A | * page 2 - page 4; claims 1-4 * | 6,7 | A61K31/353<br>A61P35/00 |
| X | WO 2006/022536 A1 (GRINDEKS JSC [LV]; KALVINS IVARS [LV]; BIRMANS ANATOLIJS [LV]) 2 March 2006 (2006-03-02)<br>* page 4 - page 12; claims 1-20 * | 1-5 | |
| Y | KR 2002 0068586 A (KOREA INST SCI & TECH [KR]; PARK EUN HEE [KR])<br>28 August 2002 (2002-08-28)<br>* abstract * | 1-5 | |
| Y | OCAKCI A ET AL: "Role of caffeic acid phenethyl ester, an active component of propolis, against NAOH-induced esophageal burns in rats",<br>INTERNATIONAL JOURNAL OF PEDIATRIC OTORHINOLARYNGOLOGY, ELSEVIER, AMSTERDAM, NL,<br>vol. 70, no. 10,<br>1 October 2006 (2006-10-01), pages 1731-1739, XP025039704,<br>ISSN: 0165-5876, DOI:<br>DOI:10.1016/J.IJPORL.2006.05.018<br>[retrieved on 2006-10-01]<br>* abstract * | 1-5 | |

TECHNICAL FIELDS
SEARCHED (IPC)

A61K
A61P

-/--

The present search report has been drawn up for all claims

| Place of search | Date of completion of the search | Examiner |
|---|---|---|
| Munich | 16 March 2011 | Kling, Isabelle |

CATEGORY OF CITED DOCUMENTS

X : particularly relevant if taken alone
Y : particularly relevant if combined with another
      document of the same category
A : technological background
O : non-written disclosure
P : intermediate document

T : theory or principle underlying the invention
E : earlier patent document, but published on, or
      after the filing date
D : document cited in the application
L : document cited for other reasons

&  : member of the same patent family, corresponding
      document

EPO FORM 1503 03.82 (P04C01)

| | Europäisches Patentamt |
|---|---|
| | European Patent Office |
| | Office européen des brevets |

## EUROPEAN SEARCH REPORT

**Application Number**

EP 10 17 3254

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CELLI NICOLA ET AL: "In vitro and in vivo stability of caffeic acid phenethyl ester, a bioactive compound of propolis", JOURNAL OF AGRICULTURAL AND FOOD CHEMISTRY, AMERICAN CHEMICAL SOCIETY, US, vol. 55, no. 9, 1 May 2007 (2007-05-01), pages 3398-3407, XP009143508, ISSN: 0021-8561, DOI: DOI:10.1021/JF0634770 [retrieved on 2007-03-30] * page 3398 - page 3399 * ----- | 1-5 | |

**TECHNICAL FIELDS SEARCHED (IPC)**

The present search report has been drawn up for all claims

| Place of search | Date of completion of the search | Examiner |
|---|---|---|
| Munich | 16 March 2011 | Kling, Isabelle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                              EP 10 17 3254

This annex lists the patent family members relating to the patent documents cited in the above-mentioned European search report.
The members are as contained in the European Patent Office EDP file on
The European Patent Office is in no way liable for these particulars which are merely given for the purpose of information.

16-03-2011

| Patent document cited in search report | Publication date | Patent family member(s) | Publication date |
|---|---|---|---|
| WO 2009071585 A1 | 11-06-2009 | AR 069521 A1 | 27-01-2010 |
| | | AU 2008333262 A1 | 11-06-2009 |
| | | CA 2706354 A1 | 11-06-2009 |
| | | CN 101951898 A | 19-01-2011 |
| | | EP 2222293 A1 | 01-09-2010 |
| | | KR 20100084688 A | 27-07-2010 |
| | | PA 8805901 A1 | 23-07-2009 |
| | | PE 10372009 A1 | 19-08-2009 |
| | | US 2010234459 A1 | 16-09-2010 |
| WO 2006022536 A1 | 02-03-2006 | LV 13450 B | 20-11-2006 |
| KR 20020068586 A | 28-08-2002 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5008441 A

- US 4481218 A

**Non-patent literature cited in the description**

- **MICHAEL J.** +)-Catechin inhibits intestinal tumor formation and suppresses focal adhesion kinase activation in the Min/+ mouse. *Cancer Research,* January 2001, vol. 61 (1), 118-125

- **SHIGEOKA ; YASUSHI ; IGISHI.** Sulindac sulfide and caffeic acid phenethyl ester suppress the motility of lung adenocarcinoma cells promoted by transforming growth factor-beta through Akt inhibition. *CANCER RESEARCH NAD CLINICAL ONCOLOGY.,* March 2004, vol. 130 (3), 146-152